# EUROPEAN PATENT SPECIFICATION

(11) **EP 0 486 483 B1**
(45) Date of publication and mention of the grant of the patent: **14.02.1996**
(21) Application number: 92300338.8
(22) Date of filing: 12.12.1988
(51) Int. Cl.: A61B 17/58, A61B 17/16

(54) **Intramedullary intertrochanteric fracture fixation appliance and fitting device**
Intramedulläre, intertrochanterische Bruckfixierungsvorrichtung sowie Montagevorrichtung
Dispositif intramedullaire pour l'ostéosynthèse intertrochantérieu et outil approprié

(30) Priority: 14.12.1987 GB 8729146; 23.06.1988 GB 8814920
(43) Date of publication of application: 20.05.1992
(62) Divisional of application: 88311746.7
(73) Proprietor: HOWMEDICA INTERNATIONAL INC., Shannon Co. Clare (IE)
(72) Inventor: Lawes, Peter, Maidenhead, Berkshire (GB); Taylor, Stephen, Basildon, Essex (GB); Adcock, Philip John, Great Wakering, Essex (GB)
(74) Representative: Bridge-Butler, Alan James

(56) References cited:
- EP-A- 0 187 283
- DE-U- 8 528 770
- DE-U- 8 620 399
- US-A- 4 622 959

## Description

This invention relates to an intramedullary intertrochanteric fracture fixation appliance which is particularly but not exclusively for use in combination with a fixation device as set forth in European Patent Application No. 88311746.7 (0 321 170).

Intramedullary intertrochanteric fracture fixation devices are known for example as shown in DE U 8 620 399 which comprise an intramedullary rod having an angulated opening to receive a femoral neck screw, which is sometimes provided in the form of a lag screw. The intramedullary rod is fitted in the intramedullary canal of the femur and the neck screw passes through an opening in the intramedullary rod, through the neck of the femur and into the head. With this kind of device it is possible to produce tension in the neck screw to pull the head and neck of the femur together and means can be provided to prevent the screw rotating both during this operation and in its final position. This can be provided by the provision of grooves in the neck screw into which a set screw can be located thus allowing the neck screw to slide but not rotate and when the pressure has been completed the set screw can be tightened up to hold the parts in a fixed position. Alternatively, some surgeons prefer to leave the set screw loose merely preventing rotation as required for the particular patient.

It is sometimes advantageous to be able to allow a limited amount of relative sliding movement between the lag screw and an intramedullary rod where weight is applied to the hip and the present invention is intended to provide a construction in which such a facility is available.

According to the present invention an intramedullary intertrochanteric fracture fixation appliance comprises an intramedullary rod (1) having an angulated opening (2) to receive a femoral neck screw (5) one end of which carries a screw thread (6) for entry into the fracture portion of the bone to be treated, said rod having a co-axial bore (3) extending into said angulated opening (2), anti-rotation means (4)(7) which can be activated to prevent rotation of said neck screw (5) in the rod (1) and characterised by adjustable means (4,7,7a,8) operable on said neck screw (5) over a predetermined length thereof and which for any lengthwise position within said predetermined length can be adjusted to define a selected portion of the predetermined length over which relative sliding movement between the rod (1) and the screw (5) is permitted in one direction while maintaining bone fragments on opposite sides of a fracture under compression, and while said anti-rotation means (4) remain active to prevent rotation of said neck screw (3).

Preferably the adjustable means comprises an adjustable stop and the anti-rotation means may also be provided by the same stop.

Preferably the anti-rotation means can also act to lock the neck screw in position.

Thus, the invention, in this form, provides three alternatives for a surgeon. He can allow a limited relative movement between the screw and the rod, locking between the two parts or relative sliding movement over a selected portion of the length of the screw.

In a preferred construction said adjustable means is provided by a set screw located in said co-axial bore and the neck screw being provided with longitudinally extending grooves or flats in the form of inclined ramps which can co-operate with the set screw to prevent rotation of the neck screw, lock it in position or provide the selected portion of the length of the screw over which there is relative sliding movement between the screw and the rod.

The open end of the bore in the rod can be provided with means to positively locate a fitting device in the manner set forth in co-pending European Patent Application No. 88311746.7 (0 321 170) the full disclosure of which is hereby incorporated in the present Application.

The invention can be performed in many ways but one embodiment will now be described by way of example and with reference to the accompanying drawings in which :-
Figure 1 is a part cross-sectional side view of a fracture fixation appliance incorporating the invention; and,
Figure 2 is a cross-sectional view of the lag screw on the line II-II of figure 1.

As shown in the drawings the intramedullary intertrochanteric fracture fixation appliance comprises an intramedullary rod 1 for introduction in to the intramedullary canal of a femur. A rod 1 has an angulated opening 2 and a co-axial bore 3 which opens into the angulated opening 2 and in which is located anti-rotation means in the form of a set screw 4.

Located in the angulated opening 2 is a femoral neck screw 5 in the formm of a lag screw, one end of which carries a coarse screw thread 6, and which is provided with four longitudinally extending ramp shaped grooves which extend over a predetermined length of the screw. As will be seen from the drawing the shallow ends 7a of the ramps, that is the deeper ends of the grooves 7 are towards the threaded end of the neck screw.

The lower end of the set screw 4 is provided with a location boss 8 which is dimensioned so that it can engage one of the grooves 7 and thus prevent rotation of the neck screw 5 when the set screw is in the position shown in the drawing. In order to receive the set screw 4 the bore 3 is screw threaded at 9 and the upper end of the bore indicated by reference numeral 10 is of a large diameter and is also screw threaded at 11. The proximal end of the intramedullary rod can be provided with two radially extending slots 12 to receive and locate a fitting device of the kind shown in co-pending European Patent Application No. 88311746.7 (0 321 170), the whole disclosure of which is hereby incorporated herein. The distal end of the rod can be provided with holes 14 and 15 to receive pins or screws to fix and locate that end of the rod in the bone.

The distal end of the neck screw 5 can be provided with driving means, for example in the form of circumferentially opposed slots 20 and is formed with a bore 21 and can be constructed as described in the co-pending European Patent Application referred to above.

As will be seen the set screw 4 acts not only as anti-rotation means but as an adjustable stop for limiting the length of sliding movement of the lag screw in relation to the rod. Thus, movement towards the left hand side of the drawing, that is in the direction of the screw threaded portion of the rod, by the screw is restricted by the set screw engaging the rising ramp in the slot 7 but, because the slot is angled away from the set screw, in the other direction, there is relative sliding movement between the parts in that direction.

All the various appliances described in European Patent Application No. 88311746.7 (0 321 170) can be used with the appliance, the subject of the present invention, and the operation can be carried out as described in that Patent Application.

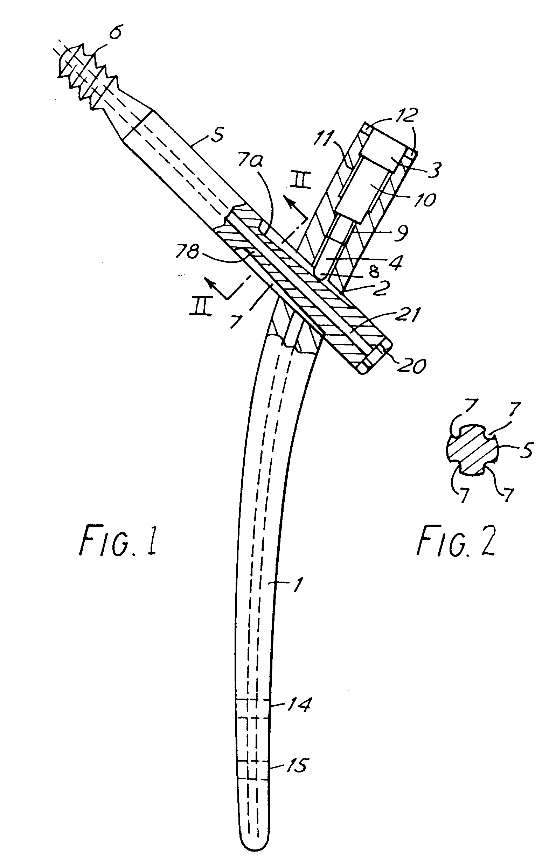

The surgeon now has three choices, when the fractured femur has been drawn together the surgeon can screw down the set screw to cause it to firmly jam in the groove 7 thereby locking the total assembly and preventing sliding. Alternatively, the surgeon may merely allow the screw to lightly engage in the groove 7 thus allowing relative sliding movement between the parts but preventing turning. The third alternative is to screw the set screw 4 down until it engages the ramp, for example as shown in Figure 1 of the drawings. In this position relative movement between the parts is allowed when compression is applied to the bone fracture. This compression is therefore maintained when the appliances described in the European Patent Application referred to above are removed. The neck screw is still free to move when body weight or muscle activity takes effect on the hip joint but the set screw 4 limits the relative movement over a selected portion of the length of the neck screw, while maintaining bone fragments on opposite sides of a fracture under compression.

## Claims

1. An intramedullary intertrochanteric fracture fixation appliance comprising an intramedullary rod (1) having an angulated opening (2) to receive a femoral neck screw (5) one end of which carries a screw thread (6) for entry into the fracture portion of the bone to be treated, said rod having a co-axial bore (3) extending into said angulated opening (2), anti-rotation means (4)(7) which can be activated to prevent rotation of said neck screw (5) in the rod (1) and characterised by adjustable means (4,7,7a,8) operable on said neck screw (5) over a predetermined length thereof and which for any lengthwise position within said predetermined length can be adjusted to define a selected portion of the predetermined length over which relative sliding movement between the rod (1) and the screw (5) is permitted in one direction while maintaining bone fragments on opposite sides of a fracture under compression, and while said anti-rotation means (4) remain active to prevent rotation of said neck screw (3).

2. An intramedullary intertrochanteric fracture fixation appliance as claimed in claim 1 characterised in that said adjustable means (4,7,7a,8) comprises an adjustable stop (4,8).

3. An intramedullary intertrochanteric fracture fixation appliance as claimed in claim 2 characterised in that said anti-rotation means (4) is also provided by said adjustable stop (4,8).

4. An intramedullary intertrochanteric fracture fixation appliance as claimed in claims 1, 2 and 3 characterised in that said anti-rotation means (4) can also act to lock the neck screw (5) in position.

5. An intramedullary intertrochanteric fracture fixation appliance as claimed in claims 1 to 4 characterised in that said adjustable means (4,7,7a,8) is provided by a set screw (4) located in said co-axial bore (3), the neck screw (5) being provided with longitudinally extending grooves (7,7a) or flats in the from of inclined ramps (7,7a) which can co-operate with the set screw (4) to prevent rotation of the neck screw (5), lock it in position, or provide the selected portion of the length of the screw (5) over which there is relative sliding movement between the screw (5) and the rod (1).

6. An intramedullary intertrochanteric fracture fixation appliance as claimed in any one of the preceding claims characterised in that the open end (1) of the bore (3) is provided with means (11,12) to positively locate a removable fitting device on the proximal end of the rod and so that said anti-rotation means (4) and said adjustable means (4,7,7a,8) to permit relative sliding movement between the rod (1) and said neck screw (5) can be operated with the fitting device in position on the rod.

7. An intramedullary intertrochanteric fracture fixation appliance as claimed in any one of the preceding claims characterised in that said anti-rotation means (4) can be operated in one position in which rotation of said neck screw 5 is prevented but axial movement is permitted in opposite directions, and another position in which rotation of said neck screw 5 is prevented but axial movement is permitted in only one direction.

## Patentansprüche

1. Intramedulläre intertrochantäre Frakturfixationseinrichtung, umfassend einen intramedullären Stab (1) mit einer angewinkelten Öffnung (2) zur Aufnahme einer Schenkelhalsschraube (5), deren eines Ende ein Schraubgewinde (6) für einen Eintritt in den Frakturteil des zu behandelnden Knochens trägt, wobei der besagte Stab eine sich in die besagte angewinkelte Öffnung (2) hinein erstreckende koaxiale Bohrung (3) und eine Verdrehsicherung (4)(7) aufweist, die betätigt werden kann, um ein Verdrehen der besagten Halsschraube (5) im Stab (1) zu verhindern, und gekennzeichnet durch eine einstellbare Einrichtung (4,7,7a,8), die man auf die besagte Halsschraube (5) über eine vorbestimmte Länge derselben einwirken lassen kann, und die für eine beliebige Längsposition innerhalb der besagten vorbestimmten Länge eingestellt werden kann, um einen ausgewählten Teil der vorbestimmten Länge festzulegen, über den eine relative Verschiebebewegung zwischen dem Stab (1) und der Schraube (5) in einer Richtung gestattet ist, während Knochenbruchstücke auf entgegengesetzten Seiten einer Fraktur unter Kompression gehalten werden, und während die besagte Verdrehsicherung (4) aktiv bleibt, um ein Verdrehen der besagten Halsschraube (3) zu verhindern.

2. Intramedulläre intertrochantäre Frakturfixationseinrichtung nach Anspruch 1, dadurch gekennzeichnet, daß die besagte einstellbare Einrichtung (4,7,7a,8) einen einstellbaren Anschlag (4,8) umfaßt.

3. Intramedulläre intertrochantäre Frakturfixationseinrichtung nach Anspruch 2, dadurch gekennzeichnet, daß die besagte Verdrehsicherung (4) ebenfalls durch den besagten einstellbaren Anschlag (4,8) bereitgestellt wird.

4. Intramedulläre intertrochantäre Frakturfixationseinrichtung nach den Ansprüchen 1, 2 und 3, dadurch gekennzeichnet, daß die besagte Verdrehsicherung (4) auch dazu dienen kann, die Halsschraube (5) in ihrer Lage zu verriegeln.

5. Intramedulläre intertrochantäre Frakturfixationseinrichtung nach den Ansprüchen 1 bis 4, dadurch gekennzeichnet, daß die besagte einstellbare Einrichtung (4,7,7a,8) durch eine in der besagten koaxialen Bohrung (3) angeordnete Stellschraube (4) bereitgestellt wird, wobei die Halsschraube (5) mit in Längsrichtung verlaufenden Nuten (7,7a) oder Abflachungen in Form von schrägen Rampen (7,7a) versehen ist, die mit der Stellschraube (4) zusammenwirken können, um ein Verdrehen der Halsschraube (5) zu verhindern, sie in ihrer Lage zu verriegeln, oder den ausgewählten Teil der Länge der Schraube (5) bereitzustellen, über den es eine relative Verschiebebewegung zwischen der Schraube (5) und dem Stab (1) gibt.

6. Intramedulläre intertrochantäre Frakturfixationseinrichtung nach einem beliebigen der vorangehenden Ansprüche, dadurch gekennzeichnet, daß das offene Ende (1) der Bohrung (3) mit einer Einrichtung (11,12) versehen ist, um eine abnehmbare Einpaßvorrichtung sicher auf dem proximalen Ende des Stabs zu fixieren, und so, daß die besagte Verdrehsicherung (4) und die besagte einstellbare Einrichtung (4,7,7a,8) zum Zulassen einer relativen Verschiebebewegung zwischen dem Stab (1) und der besagten Halsschraube (5) betätigt werden kann, wenn sich die Einpaßvorrichtung in ihrer Lage auf dem Stab befindet.

7. Intramedulläre intertrochantäre Frakturfixationseinrichtung nach einem beliebigen der vorangehenden Ansprüche, dadurch gekennzeichnet, daß die besagte Verdrehsicherung (4) in eine Position gebracht werden kann, in der ein Verdrehen der besagten Halsschraube (5) verhindert wird, jedoch eine axiale Bewegung in entgegengesetzten Richtungen zugelassen ist, und in eine andere Position, in der ein Verdrehen der besagten Halsschraube (5) verhindert wird, jedoch eine axiale Bewegung in nur einer Richtung zugelassen ist.

## Revendications

1. Instrument intramédullaire pour l'ostéosynthèse intertrochantérienne comprenant une tige intramédullaire (1) ayant une ouverture inclinée (2) pour recevoir une vis pour col du fémur (5), dont une extrémité porte un filetage de vis (6) pour entrer à l'intérieur de la partie de la fracture de l'os à traiter, ladite tige ayant un alésage coaxial (3) pénétrant à l'intérieur de ladite ouverture inclinée (2); des moyens anti-rotation (4), (7) qui peuvent être activés pour empêcher la rotation de ladite vis pour col (5) dans la tige (1), et caractérisé par des moyens ajustables (4, 7, 7a, 8) pouvant agir sur ladite vis pour col (5) suivant une longueur prédéterminée de celle-ci et qui, pour toute position dans le sens de la longueur à l'intérieur de ladite longueur prédéterminée, peuvent être ajustés pour définir une partie sélectionnée de la longueur prédéterminée, sur laquelle un mouvement de coulissement relatif entre la tige (1) et la vis (5) est permis dans une direction, tout en maintenant les fragments d'os sur les côtés opposés d'une fracture sous compression, et tandis que lesdits moyens anti-rotation (4) restent actifs pour empêcher la rotation de ladite vis pour col (3).

2. Instrument intramédullaire pour l'ostéosynthèse intertrochantérienne selon la revendication 1, caractérisé en ce que lesdits moyens ajustables (4, 7, 7a, 8) comprennent une butée ajustable (4, 8).

3. Instrument intramédullaire pour l'ostéosynthèse intertrochantérienne selon la revendication 2, caractérisé en ce que lesdits moyens anti-rotation (4) sont également pourvus de ladite butée ajustable (4, 8).

4. Instrument intramédullaire pour l'ostéosynthèse intertrochantérienne selon les revendications 1, 2 et 3, caractérisé en ce que lesdits moyens anti-rotation (4) peuvent également agir pour bloquer la vis pour col (5) en position.

5. Instrument intramédullaire pour l'ostéosynthèse intertrochantérienne selon les revendications 1 à 4, caractérisé en ce que lesdits moyens ajustables (4, 7, 7a, 8) sont fournis par une vis de réglage (4) située dans ledit alésage coaxial (3), la vis pour col (5) étant pourvue de rainures (7, 7a) s'étendant longitudinalement ou de parties plates sous la forme de rampes inclinées (7, 7a) qui peuvent coopérer avec la vis de réglage (4) pour empêcher la rotation de la vis pour col (5), la bloquer en position, ou fournir la partie sélectionnée de la longueur de la vis (5) sur laquelle il y a un mouvement de coulissement relatif entre la vis (5) et la tige (1).

6. Instrument intramédullaire pour l'ostéosynthèse intertrochantérienne selon l'une quelconque des revendications précédentes, caractérisé en ce que l'extrémité ouverte (1) de l'alésage (3) est pourvue de moyens (11, 12) pour localiser de façon sûre un dispositif de mise en place amovible sur l'extrémité proximale de la tige, et qu'ainsi lesdits moyens anti-rotation (4) et lesdits moyens ajustables (4, 7, 7a, 8), pour permettre un mouvement de coulissement relatif entre la tige (1) et ladite vis pour col (5), puissent fonctionner avec le dispositif de mise en place en position sur la tige.

7. Instrument intramédullaire pour l'ostéosynthèse intertrochantérienne selon l'une quelconque des revendications précédentes, caractérisé en ce que lesdits moyens anti-rotation (4) peuvent fonctionner dans une position dans laquelle la rotation de ladite vis pour col 5 est empêchée, mais le mouvement axial est permis dans des directions opposées, et dans une autre position dans laquelle la rotation de ladite vis pour col (5) est empêchée, mais le mouvement axial est permis seulement dans une direction.
